# EUROPEAN PATENT SPECIFICATION

(11) **EP 4 215 229 B1**
(45) Date of publication and mention of the grant of the patent: **16.04.2025**
(21) Application number: 21885972.6
(22) Date of filing: 19.10.2021
(51) Int. Cl.: A61M 5/315

(54) **SYRINGE GASKET AND SYRINGE**
SPRITZENDICHTUNG UND SPRITZE
CYLINDRE DE SERINGUE ET SERINGUE

(30) Priority: 27.10.2020 JP 2020179888
(43) Date of publication of application: 26.07.2023
(73) Proprietor: Taisei Kako Co., Ltd., Kita-ku Osaka-shi Osaka 531-0072 (JP); Sumitomo Rubber Industries, Ltd., Kobe-shi, Hyogo 651-0072 (JP)
(72) Inventor: HORITA, Taiji, Ibaraki-shi, Osaka 567-0054 (JP); KUROKI, Makoto, Ibaraki-shi, Osaka 567-0054 (JP); UMEZAKI, Masaya, Ibaraki-shi, Osaka 567-0054 (JP); NAKANO, Hiroaki, Kobe-shi, Hyogo 651-0072 (JP); TANAKA, Yuki, Kobe-shi, Hyogo 651-0072 (JP)
(74) Representative: DTS Patent- und Rechtsanwälte PartmbB
(86) International application number: PCT/JP2021/038505
(87) International publication number: WO 2022/091853

(56) References cited:
- WO-A1-2014/028936
- WO-A1-2019/031028
- JP-A- 2020 522 406
- JP-A- 2020 523 070
- US-A1- 2003 233 075
- US-A1- 2011 224 610
- US-A1- 2015 217 059

## Description

### CROSS-REFERENCE TO RELATED APPLICATION

This application claims priority to Japanese Patent Application No. 2020-17988.

### FIELD

The present invention relates to a syringe gasket having an element therein and a syringe including the syringe gasket.

### BACKGROUND

Conventionally, there is known a gasket (piston) configured to slide in a cylindrical barrel (cartridge body) while maintaining liquid-tightness so as to prevent leakage of a medical solution in the barrel (see Patent Literature 1). The gasket has elasticity in order to ensure the liquid-tightness by being in close contact with an inner peripheral surface of the barrel. Also, the gasket has an element (microchip) therein that stores data on at least one of the date and time of the production (date and time when the barrel is filled with a medical solution). It is possible to check at least one of the date and time date and time when the barrel is filled with a medical solution by reading the data of the element, thereby facilitating, for example, quality control of the medical solution filled in the barrel.

There is a case where, when the gasket is inserted into the barrel, the elastic deformation of a portion of the gasket that comes into close contact with the barrel is hardly made due to, for example, the arrangement position of an element such as a microchip, thereby increasing the resistance (sliding resistance) at the time of sliding the gasket in the barrel.

### CITATION LIST

### Patent Literature

Patent Literature 1: JP 2017-94151 A
Patent Literature 2: US2015217059A1 relates to a plunger comprising an embedded RFID element.

### SUMMARY

### Technical Problem

It is therefore an object of the present invention to provide a syringe gasket capable of suppressing a sliding resistance with a barrel even it has an element therein, and a syringe including the syringe gasket.

### Solution to Problem

A syringe gasket according to the present invention is a syringe gasket configured to be connected to a distal end of a plunger rod, while being disposed in a cylindrical barrel, the syringe gasket including: a first member having a large diameter part and a small diameter part, the large diameter part being in close contact with an entire area of an inner peripheral surface of the barrel in a circumferential direction when the syringe gasket is inserted into the barrel, the small diameter part being located adjacent to the large diameter part and having a smaller diameter than the large diameter part; a second member configured to be connected to the first member from a side of the small diameter part in an axial direction that is a direction in which the large diameter part and the small diameter part are aligned with each other; and an element disposed between the first member and the second member, the first member and the second member having elasticity, the first member having a recess that is recessed from an end surface on the side of the second member toward the large diameter part in the axial direction, the recess having a bottom that is recessed to reach a boundary position between the large diameter part and the small diameter part in the axial direction or a certain position on the side of the second member relative to the boundary position, the second member having a projection that projects toward the first member and is configured to fit into the recess with the element disposed between the recess and the projection, and having a screw hole extending from an end surface of the second member on an opposite side to the first member toward the first member to reach an inside of the projection in the axial direction to allow a screw part provided at the distal end of the plunger rod to be screwed thereinto.

The syringe gasket can be configured such that the small diameter part has an annular projection that projects radially outward at a position with a distance from the large diameter part in the axial direction, while extending through the entire area in the circumferential direction, the annular projection is in close contact with an entire area of the inner peripheral surface of the barrel in the circumferential direction when the syringe gasket is inserted into the barrel, the element is disposed between the large diameter part and the annular projection in the axial direction, and the screw hole extends at least to a position of the annular projection in the axial direction.

Further, a syringe according to the present invention includes: a syringe body having a cylindrical barrel: the syringe gasket of any one of the above being disposed in the barrel; and a plunger rod extending along a central axis of the barrel and having a screw part that is provided in a distal end of the plunger rod and configured to be threaded into the screw hole of the syringe gasket.

### BRIEF DESCRIPTION OF DRAWINGS

Fig. 1 is a view of a syringe according to this embodiment in a state where a syringe body and a plunger rod are separated from each other.
Fig. 2 is a cross sectional view of a gasket, its peripheral portion, and a proximal end of the syringe body in a state where the syringe body and the plunger rod are separated from each other.
Fig. 3 is a cross sectional view of the gasket in an exploded state.
Fig. 4 is an exploded perspective view of the gasket seen from the proximal end side of the gasket.
Fig. 5 is an exploded perspective view of the gasket seen from the distal end side of the gasket.
Fig. 6 is a view for explaining a method for producing the gasket.
Fig. 7 is a cross sectional view of the gasket, its peripheral portion, and a base part of the syringe body in a state where the plunger rod is inserted into the syringe body.

### DESCRIPTION OF EMBODIMENTS

A syringe gasket according to this embodiment is a syringe gasket configured to be connected to a distal end of a plunger rod, while being disposed in a cylindrical barrel, the syringe gasket including: a first member having a large diameter part and a small diameter part, the large diameter part being in close contact with an entire area of an inner peripheral surface of the barrel in a circumferential direction when the syringe gasket is inserted into the barrel, the small diameter part being located adjacent to the large diameter part and having a smaller diameter than the large diameter part; a second member configured to be connected to the first member from a side of the small diameter part in an axial direction that is a direction in which the large diameter part and the small diameter part are aligned with each other; and an element disposed between the first member and the second member, the first member and the second member having elasticity, the first member having a recess that is recessed from an end surface on the side of the second member toward the large diameter part in the axial direction, the recess having a bottom that is recessed to reach a boundary position between the large diameter part and the small diameter part in the axial direction or a certain position on the side of the second member relative to the boundary position, the second member having a projection that projects toward the first member and is configured to fit into the recess with the element disposed between the recess and the projection, and having a screw hole extending from an end surface of the second member on an opposite side to the first member toward the first member to reach an inside of the projection in the axial direction to allow a screw part provided at the distal end of the plunger rod to be screwed thereinto.

Accordingly, the element is disposed at a position displaced in the axial direction (i.e., between the recess of the first member and the projection of the second member) relative to the large diameter part that is brought into close contact with the inner peripheral surface of the barrel by the elasticity when the syringe gasket is disposed within the barrel. Thus, it is possible to ensure ease of elastic deformation of the large diameter part in a radial direction. Also, the screw hole extending to the inside of the projection is provided in the second member to minimize the thickness in the radial direction of a part in which two members overlap with each other in the radial direction in the syringe gasket (i.e., a portion with the projection of the second member fitting into the recess of the first member). Thus, it is possible to ensure ease of elastic deformation of this portion. These configurations can suppress sliding resistance when the syringe gasket slides in the barrel.

The syringe gasket can be configured such that the small diameter part has an annular projection that projects radially outward at a position with a distance from the large diameter part in the axial direction, while extending through the entire area in the circumferential direction, the annular projection is in close contact with an entire area of the inner peripheral surface of the barrel in the circumferential direction when the syringe gasket is inserted into the barrel, the element is disposed between the large diameter part and the annular projection in the axial direction, and the screw hole extends at least to a position of the annular projection in the axial direction.

According to this configuration, the syringe gasket is in close contact with the inner peripheral surface of the barrel at two positions (i.e., the position of the large diameter part and the position of the annular projection) when the syringe gasket is inserted into the barrel. Thus, the liquid-tightness between the barrel and the gasket can be sufficiently ensured. Moreover, the screw hole extends at least to the position at which the annular projection is formed in the syringe gasket to thereby suppress the thickness in the radial direction of this portion (that is, in order to ensure ease of elastic deformation of this portion). Thus, it is possible to effectively suppress the increase in the sliding resistance due to the increase in parts in close contact with the inner peripheral surface of the barrel.

Further, a syringe according to this embodiment includes: a syringe body having a cylindrical barrel: the syringe gasket of any one of the above being disposed in the barrel; and a plunger rod extending along a central axis of the barrel and having a screw part that is provided in a distal end of the plunger rod and configured to be threaded into the screw hole of the syringe gasket.

According to this configuration, the element is disposed at a position displaced in the axial direction (i.e., between the recess of the first member and the projection of the second member) relative to the large diameter part that is in close contact with the inner peripheral surface of the barrel due to the elasticity. Thereby, it is possible to ensure ease of elastic deformation (flexibility) of the large diameter part in a radial direction. Also, the screw hole extending to the inside of the projection is provided in the second member to minimize the thickness in the radial direction of a part in which the two members overlap with each other in the radial direction in the syringe gasket (i.e., a portion with the projection of the second member fitting into the recess of the first member). Thereby, it is possible to ensure ease of elastic deformation of this portion. These configurations can suppress the sliding resistance when the syringe gasket slides in the barrel.

As described above, according to this embodiment, it is possible to provide a syringe gasket capable of suppressing sliding resistance even it has an element therein, and a syringe including the syringe gasket.

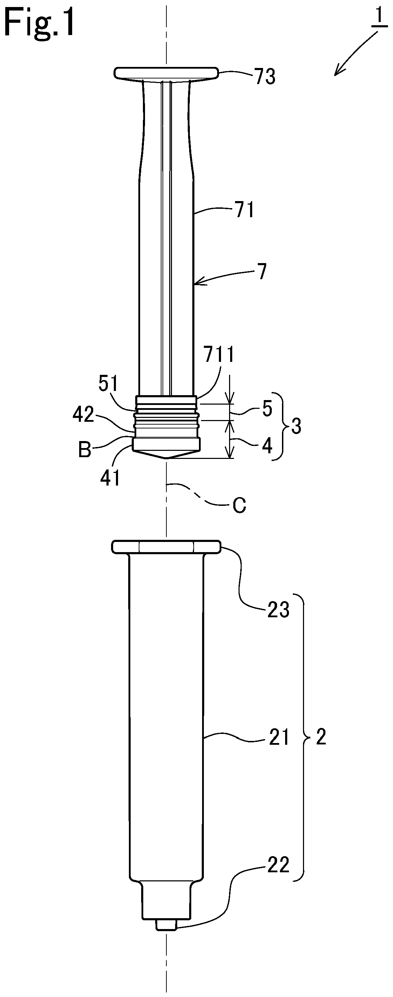

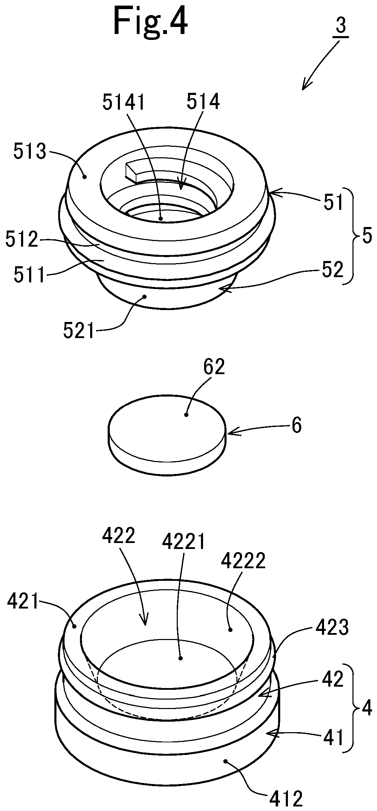

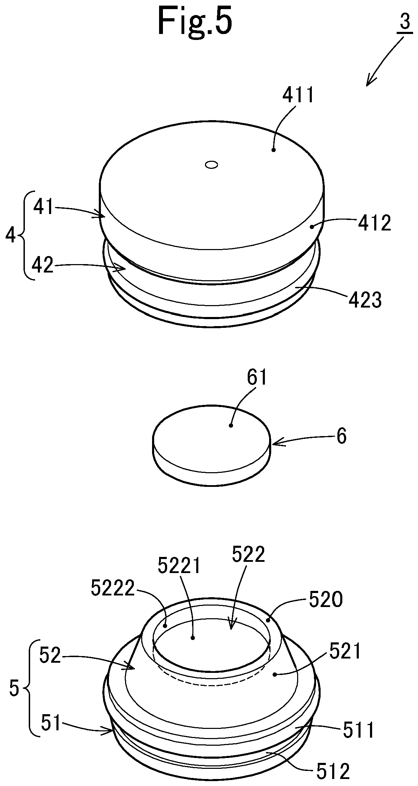

Hereinafter, an embodiment of the present invention will be described with reference to Fig. 1 to Fig. 7.

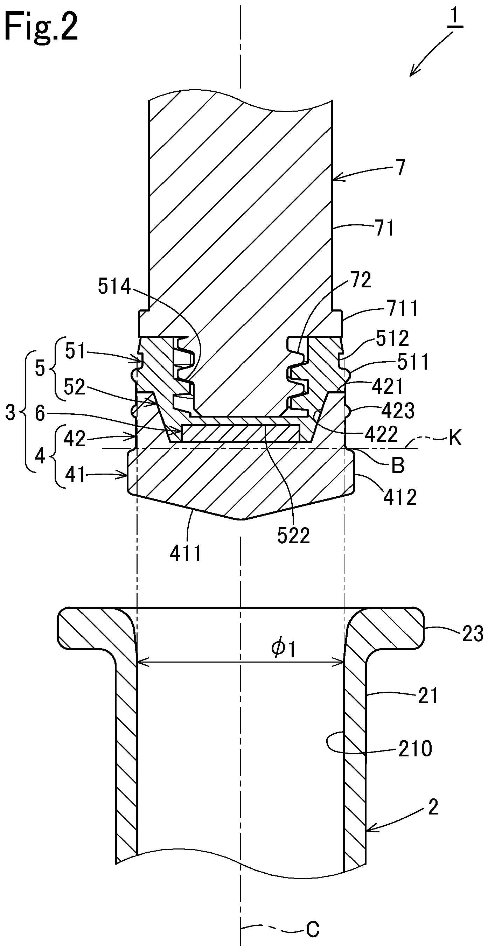

As shown in Fig. 1 and Fig. 2, the syringe according to this embodiment includes a syringe body 2 having a cylindrical barrel 21, a gasket (syringe gasket) 3 to be inserted into the barrel 21, and a plunger rod 7 extending in a direction of a central axis C of the barrel 21 and having a distal end to which a gasket 3 is connected.

The syringe body 2 includes a barrel (trunk) 21, a discharge part 22 extending from one end (i.e., distal end) in the direction of the central axis C of the barrel 21, and a flange 23 disposed at the other end (i.e., proximal end) in the direction of the central axis C of the barrel 21. The syringe body 2 is rigid and formed by, for example, glass or a hard resin (e.g., plastic) through which the inside is visible. Note that, hereinafter, one side (i.e., lower side in Fig. 1) in the direction of the central axis C will be referred to as a distal side, and the other side (i.e, upper side in Fig. 1) in the central axis C direction will be referred to as a proximal side.

The cylindrical barrel 21 has an inner peripheral surface 210 having an inner diameter (diameter) constant at every position in the direction of the central axis C. The distal end of the barrel 21 except for the discharge part 22 is closed, and the proximal end of the barrel 21 is open (see Fig. 2). The syringe body 2 of this embodiment has a nominal volume of 5 mL, and an inner diameter ϕ1 of the barrel 21 is, for example, approximately 11.6 mm to 12.5 mm.

The discharge part 22 projects in a cylindrical shape from the distal end of the barrel 21 and is a part with which a hub of a needle tube or a connector can be fitted (mounted).

The flange 23 is a plate-shaped part to which a fingers are hooked when the plunger rod 7 is relatively moved to the syringe body 2 (i.e., barrel 21) in the direction of the central axis C, and extends from the barre 21 along a surface (i.e., virtual surface) orthogonal to the central axis C.

The plunger rod 7 includes a rod body 71 extending in the direction of the central axis C, and a male screw part (screw part) 72 extending from the distal end of the rod body 71 in the direction of the central axis C and having a male screw on an outer peripheral surface. The plunger rod 7 also includes a flange 73 disposed at the proximal end of the rod body 71 and extending along a surface (i.e., virtual surface) orthogonal to the central axis C. The plunger rod 7 is rigid and formed by, for example, a hard resin (e.g., plastic).

The rod body 71 includes a plate-shaped pressing part 711 located at the distal end and extends in a direction orthogonal to the central axis C. The pressing part 711 is a part configured to press the gasket 3 in the barrel 21. The pressing part 711 of this embodiment has a disc shape with a diameter (outer diameter) slightly larger than a part of the gasket 2 which abuts the pressing part 711 and slightly smaller than the inner diameter ϕ1 of the barrel 21.

The male screw part 72 extends from a center of the disc-shaped pressing part 711 in the direction of the central axis C.

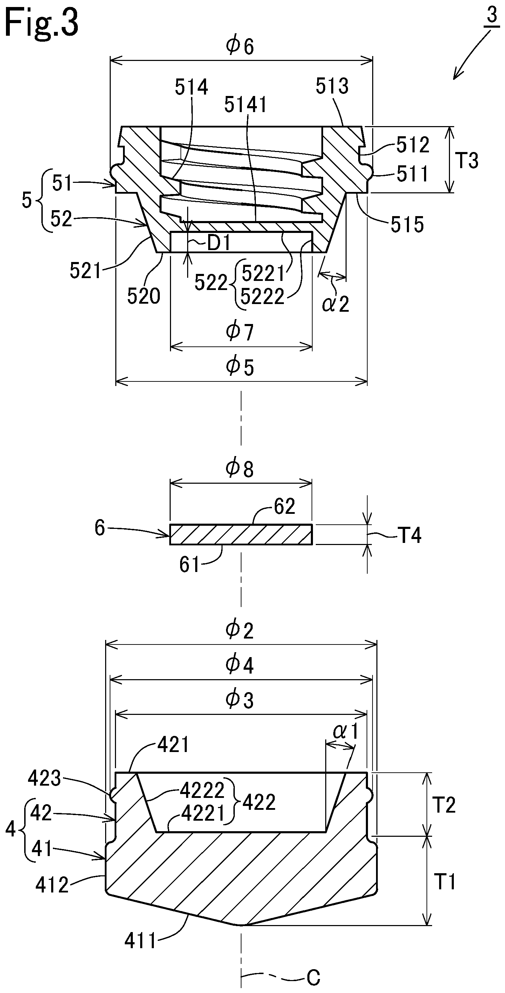

The gasket 3 is a column-shaped member capable of sliding in the barrel 21 while maintaining the liquid-tightness between the gasket 3 and the inner peripheral surface 210 of the barrel 21. The gasket 3 of this embodiment is composed of a plurality of members. Specifically, as shown in Fig. 3 to Fig. 5, the gasket 3 includes a first member 4, a second member 5 to be connected to the first member 4 from the proximal side of the first member 4, and an RF tag (element) 6 disposed between the first member 4 and the second member 5. The first member 4 and the second member 5 are aligned with each other in the direction of the central axis C and have elasticity.

The first member 4 and the second member 5 each are made of an elastic material and typically, made of rubber or a thermoplastic elastomer. More specifically, the first member 4 and the second member 5 can be made of, for example, butyl rubber, isoprene rubber, butadiene rubber, halogenated butyl rubber, ethylene propylene terpolymer, and silicone rubber, but are not limited to these examples. Also, the first member 4 and the second member 5 can be made of the same material or can be made of different materials.

The first member 4 includes a large diameter part 41 that is in close contact with an entire area of the inner peripheral surface 210 of the barrel 21 in a circumferential direction when the gasket 3 is inserted into the barrel 21, and a small diameter part 42 that is located adjacent to the large diameter part 41 in the direction of the central axis C and has a smaller diameter (outer diameter) than the large diameter part 41. The first member 4 of this embodiment is made of butyl rubber.

The large diameter part 41 is a column-shaped part having a center on the central axis C and has a surface 411 that faces the distal side to serve as a distal end surface of the gasket 3. In the large diameter part 41 of this embodiment, the distal end surface 411 has a conical shape, and a peripheral surface 412 has a cylindrical surface shape. An outer diameter ϕ2 of the large diameter part 41 is larger than the inner diameter ϕ1 of the barrel 21 (see Fig. 2). Thus, when the gasket 3 is inserted into the barrel 21, the large diameter part 41 as a whole is elastically deformed (i.e., elastically compressed) radially inward (i.e., toward the central axis C), thereby causing the peripheral surface 412 of the large diameter part 41 to come into close contact with the inner peripheral surface 210 of the barrel 21.

The outer diameter ϕ2 of the large diameter part 41 of this embodiment is, for example, approximately 11.8 mm to 13.8 mm. The size (thickness) T1 of the large diameter part 41 in the direction of the central axis C is, for example, approximately 2.3 mm to 4.9 mm.

The small diameter part 42 is a part extending from the large diameter part 41 to the proximal side and having a columnar-shaped appearance having a center on the central axis C. An outer diameter (diameter) ϕ3 of the small diameter part 42 is smaller than an outer diameter (diameter) ϕ2 of the large diameter part 41. The small diameter part 42 has a recess 422 recessed from an end surface 421 on the proximal side (that is, on the side of the second member 5) toward the distal side, that is, toward the large diameter part 41 in the direction of the central axis C. The small diameter part 42 also has a first annular projection (annular-shaped projection) 423 that projects radially outward at a position with a distance from the large diameter part 41 on the proximal side in the direction of the central axis C, and extends through an entire area in the circumferential direction.

In the small diameter part 42 of this embodiment, the outer diameter ϕ3 is set to be constant at every position in the direction of the central axis C except for the position at which the first annular projection 423 is provided, and is, for example, approximately 11.1 mm to 12.8 mm. The outer diameter ϕ3 is set to have such a size as to cause no contact with the inner peripheral surface 210 of the barrel 21 even in a state where the gasket 3 is inserted into the barrel 21.

The recess 422 is recessed from the end surface 421 of the small diameter part 42 to a boundary position B between the large diameter part 41 and the small diameter part 42 or a certain position closer to the proximal side than the boundary position B. Specifically, the recess 422 is recessed to a certain position on the proximal side of a virtual surface K including the boundary position between the large diameter part 41 and the small diameter part 42 on the surface of the gasket 3 (in the example shown in Fig. 2, a virtual surface extending in the direction orthogonal to the central axis C and including the boundary position on the surface of the gasket 3). That is, the boundary position B in this embodiment means not only the boundary position between the large diameter part 41 and the small diameter part 42 on the surface of the gasket 3 but also a position defined by the virtual surface K including the boundary position and extending in the direction orthogonal to the central axis C. The recess 422 of this embodiment is recessed from the end surface 421 to the boundary position B and is a recess having a truncated cone-shape. Specifically, in the recess 422, a bottom surface 4221 is a circular surface extending in the direction orthogonal to the central axis C, and an inner peripheral surface (i.e., surface surrounding the central axis C) 4222 connected to a peripheral edge of the bottom surface 4221 has a tapered shape with an inner diameter gradually decreasing toward the bottom surface 4221.

A depth of the recess 422 of this embodiment, that is, a distance from the end surface 421 to the bottom surface 4221 in the direction of the central axis C is approximately 2.8 mm to 4.1 mm. An angle α1 of the inner peripheral surface 4222 relative to the central axis C is larger than 0° (not vertical), preferably 5° or more, more preferably 10° or more. The angle α1 is limited by the outer diameter (ϕ3) of the small diameter part 42 and an outer diameter (ϕ8) of an RF tag 6 to be described later, and thus the limited angle is accordingly adopted. The larger the angle α1, the easier the arrangement of the RF tag 6 at the time of assembly (i.e., at the time of production) of the gasket 3. Further, joining of the first member 4 to the second member 5 can be easily performed and become strong.

The first annular projection 423 is in close contact with an entire area of the inner peripheral surface 210 of the barrel 21 in the circumferential direction when the gasket 3 is inserted into the barrel 21. In the small diameter part 42, an outer diameter ϕ4 of a part in which the first annular projection 423 is located is larger than the outer diameter ϕ3 of the other part in the direction of the central axis C and smaller than the outer diameter ϕ2 of the large diameter part 41. The outer diameter ϕ4 of a part in which the first annular projection 423 is provided in the small diameter part 42 of this embodiment is, for example, approximately 11.7 mm to 13.36 mm.

The second member 5 includes a columnar-shaped base part 51, and a projection 52 that projects from the base part 51 toward the distal side (i.e., toward the first member 4) and fits into the recess 422 with the RF tag 6 located between the projection 52 and the recess 422 of the first member 4. The second member 5 of this embodiment is made of butyl rubber.

The base part 51 includes: a second annular projection 511 that projects radially outward at a position with a distance from the first annular projection 423 of the first member 4 on the proximal side in the direction of the central axis C, and extends through an entire area in the circumferential direction; and an annular recess 512 that is provided at a position adjacent to the second annular projection 511 on the proximal side in the direction of the central axis C. The annular recess 512 extends through an entire area in the circumferential direction. In the base part 51 of this embodiment, a part on the proximal side of the annular recess 512 has a tapered shape with the outer diameter gradually decreasing toward the proximal side. The part on the proximal side of the annular recess 512 is preferably smaller than the inner diameter ϕ1 of the barrel 21.

Also, the base part 51 includes a screw hole 514 extending from an end surface on the proximal side (i.e., end surface opposite to the first member 4) 513 toward the distal side, that is, toward the first member 4. The end surface 513 on the proximal side of the base part 51 of this embodiment serves as an end surface on the proximal side of the gasket 3.

An outer diameter ϕ5 of the base part 51 is the same or substantially the same as the outer diameter ϕ3 of the small diameter part 42, and is, for example, approximately 11.1 mm to 12.8 mm. A size (thickness) T3 of the base part 51 in the direction of the central axis C is, for example, approximately 2.9 mm to 3.53 mm.

The second annular projection 511 is in close contact with an entire area of the inner peripheral surface 210 of the barrel 21 in the circumferential direction when the gasket 3 is inserted into the barrel 21. In the base part 51, an outer diameter ϕ6 of a part in which the second annular projection 511 is provided is larger than the outer diameter ϕ5 of the other part in the direction of the central axis C and smaller than the outer diameter ϕ2 of the large diameter part 41 of the first member 4. In the base part 51 of this embodiment, the outer diameter ϕ6 of a part in which the second annular projection 511 is provided is the same or substantially the same as the outer diameter ϕ4 of the part in which the first annular projection 423 of the first member 4 is provided. In the base part 51 of this embodiment, the outer diameter ϕ6 of the part in which the second annular projection 511 is provided is, for example, approximately 11.7 mm to 13.36 mm.

The screw hole 514 is a hole, into which the male screw part 72 of the plunger rod 7 is screwed, and has a female screw on its inner peripheral surface surrounding the central axis C. The screw hole 514 extends to the inside of the projection 52 of the second member 5 in the direction of the central axis C. The screw hole 514 of this embodiment extends from the end surface 513 of the base part 51 to the position of the first annular projection 423 of the first member 4 in the direction of the central axis C. More specifically, the screw hole 514 extends from the end surface 513 toward the distal side so as to have the position of a bottom surface 5141 of the screw hole 514 coinciding with an end edge position on the distal side of the first annular projection 423 in the direction of the central axis C. A hole diameter of the screw hole 514 is set so that a gap is formed between the inner peripheral surface of the screw hole 514 and the male screw part 72 of the plunger rod 7 (see Fig. 2).

The projection 52 has a distal end recess 522 located at the distal end into which the RF tag 6 is fitted. The projection 52 has a shape corresponding to the recess 422 of the first member 4. Specifically, the projection 52 has a truncated cone shape with its outer diameter gradually decreasing toward the distal side, that is, has a tapered outer peripheral surface 521, and has a distal end surface 520 having the distal end recess 522 recessed toward the proximal side. The distal end surface 520 is a circular-shaped surface extending in the direction orthogonal to the central axis C and has the same diameter as the bottom surface 4221 of the recess 422 of the first member 4. The shape of the projection 52 of this embodiment, that is, the shape of each of the distal end surface 520, the distal end recess 522, the tapered outer peripheral surface 521, etc. is formed in conformity with the shape of the recess 422 and the shape of the RF tag 6 respectively by a material (i.e., material for forming the second member 5) that is solidified after being injected into the recess 422 of the first member 4 with the RF tag 6 disposed inside the recess 422. Specific descriptions will be given below.

First, the first member 4 is formed. The aforementioned elements 41, 42, 421, 423, etc., are formed in the first member 4. Note that the method for forming the first member 4 is not limited.

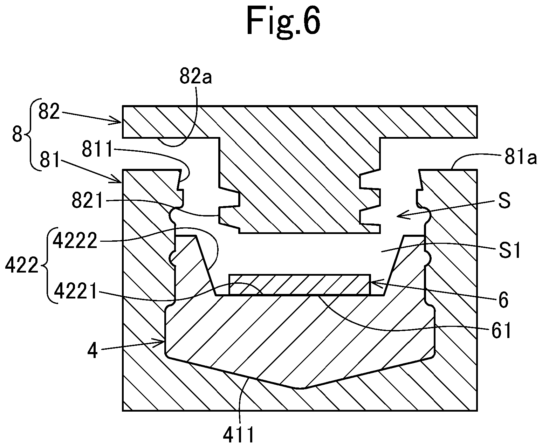

Next, as shown in Fig. 6, the first member 4 is disposed on a cavity 811 that is formed in a lower mold 81 out of a pair of upper and lower molds 8 (i.e., lower mold 81 and upper mold 82). The cavity 811 of the lower mold 81 is a columnar-shaped recess extending downward from an upper end surface 81a of the lower mold 81 and has a shape corresponding to the outer peripheral surface and the distal end surface 411, of the gasket 3. Here, the upper mold 82 out of the pair of molds 8 includes a projection 821 extending downward a lower end surface 82a. The projection 821 has a shape corresponding to the screw hole 514 of the gasket 3 (the second member 5). In these lower mold 81 and upper mold 82, when the lower mold 81 and the upper mold 82 are closed (overlapped with each other) to allow the projection 821 to be inserted along the center of the cavity 811, a space S having a shape corresponding to the gasket 3 is formed between the lower mold 81 and the upper mold 82 (i.e., inside the pair of molds 8).

The first member 4 disposed on the bottom of the cavity 811 is oriented such that the distal end surface 411 is located at the lower end of the first member 4 and the recess 422 is located at the upper end of the first member 4. With the first member 4 held in this state, the RF tag 6 is disposed in the recess 422 in a state where a surface on one side (i.e., distal side surface) faces the bottom surface 4221.

Subsequently, a material for the second member 5 is injected into the cavity 811 (i.e., the space S defined in the inside of the pair of molds 8 except for an area in which the first member 4 is disposed), while the lower mold 81 and the upper mold 82 are closed. When the material is solidified by, for example, heating or vulcanization with the area S1 filled with the material, the second member 5 having a shape corresponding to the area S1 and joined to the first member 4 is formed, that is, the gasket 3 is formed.

When the material for the second member 5 is injected into the area S1 as described above, the inside of the recess 422 with the RF tag 6 disposed on the bottom surface 4221 is filled with the material, and then the material is solidified. Thereby, the distal end recess 522 having a shape in conformity to the shape of the RF tag 6 is formed in the distal end surface 520 of the projection 52 of the second member 5.

The projection amount of the projection 52 of this embodiment, that is, the distance from the distal end surface 515 of the base part 51 to the distal end surface 520 of the projection 52 in the direction of the central axis C is the same as the depth of the recess 422 of the first member 4, and is approximately 2.8 mm to 4.1 mm. Also, an angle α2 of the outer peripheral surface 521 relative to the central axis C is the same as the angle of the inner peripheral surface 4222 of the recess 422 relative to the central axis C, and is 0° or more (not vertical), preferably 5° or more, more preferably 10° or more. The angle α2 is limited by the outer diameter (ϕ3) of the small diameter part 42 and the outer diameter (ϕ8) of the RF tag 6 to be described later, and thus the limited angle is accordingly adopted.

The distal end recess 522 is recessed to correspond to the shape of the RF tag 6. The distal end recess 522 of this embodiment is recessed so that the distal end surface 520 is flush or substantially flush with the distal side surface 61 of the plate-shaped RF tag 6 with the RF tag 6 fitted in the distal end recess 522. Specifically, the distal end recess 522 has a circular-shaped bottom surface 5221 extending in the direction orthogonal to the central axis C and an inner peripheral surface 5222 having an inner diameter ϕ7 that is constant at every position in the direction of the central axis C. A depth D1 of the distal end recess 522 of this embodiment (i.e, distance in the direction of the central axis C from the distal end surface 520 to the bottom surface 5221) is approximately 0.05 mm to 2.2 mm.

In the first member 4 and the second member 5 configured as above, with the RF tag 6 being disposed in the area surrounded by the bottom surface 4221 of the recess 422 of the first member 4 and the distal end recess 522 provided in the projection 52 of the second member 5, the bottom surface 4221 of the recess 422 in the first member 4 and the distal end surface 520 of the projection 52 in the second member 5 are adhered (joined) to each other; the inner peripheral surface 4222 of the recess 422 in the first member 4 and the outer peripheral surface 521 of the projection 52 in the second member 5 are adhered (joined) to each other; and the end surface 421 on the proximal side in the first member 4 and the distal end surface 515 of the base part 51 in the second member 5 are adhered (joined) to each other.

Further, in the gasket 3, the depth of the recess 422 of the first member 4 (that is, the projection amount of the projection 52 of the second member 5) and the depth D1 of the distal end recess 522 in the projection 52 of the second member 5 are set so that the RF tag 6 is located between the large diameter part 41 and the first annular projection 423 of the first member 4 in the direction of the central axis C (see Fig. 2).

The RF tag 6 is capable of storing (recording) information about the liquid (e.g., medical solution) to be, for example, sealed in the syringe 1, information about the date and time of sealing, and the production record, and information of the sealed medical solution, and allows the aforementioned information to be read out and written in by, for example, a reader-writer from the outside of the syringe with the liquid sealed therein. This RF tag has a plate shape extending in the direction orthogonal to the central axis C, and the RF tag 6 of this embodiment has a disc shape. The RF tag 6 is harder than the first member 4 and the second member 5. That is, the hardness of the RF tag 6 is higher than the first member 4 and the second member 5.

In the RF tag 6, the outer diameter ϕ8 is preferably 55% or more of the diameter of the bottom surface 4221 of the recess 422 in the first member 4. Thereby, displacement (movement) of the RF tag 6 at the time of producing the gasket 3 can be suppressed.

Also in terms of the sliding resistance (i.e., in terms of preventing the degradation of slidability), it is preferable to select at least one of the configurations including: the configuration in which the outer diameter ϕ8 of the RF tag 6 is 70% or less of the outer diameter ϕ2 of the large diameter part 41 of the first member 4; the configuration in which the outer diameter ϕ8 of the RF tag 6 is 75% or less of the outer diameter (i.e., outer diameter of the small diameter part 42) ϕ3 at the part in which the RF tag 6 of the gasket 3 is disposed in the direction of the central axis C; and the configuration in which the outer diameter ϕ8 of the RF tag 6 is 50% or less of the inner diameter ϕ1 of the barrel 21.

The outer diameter (diameter) ϕ8 of the RF tag 6 is, for example, approximately 8 mm, and the thickness T4 thereof is, for example, approximately 1 mm.

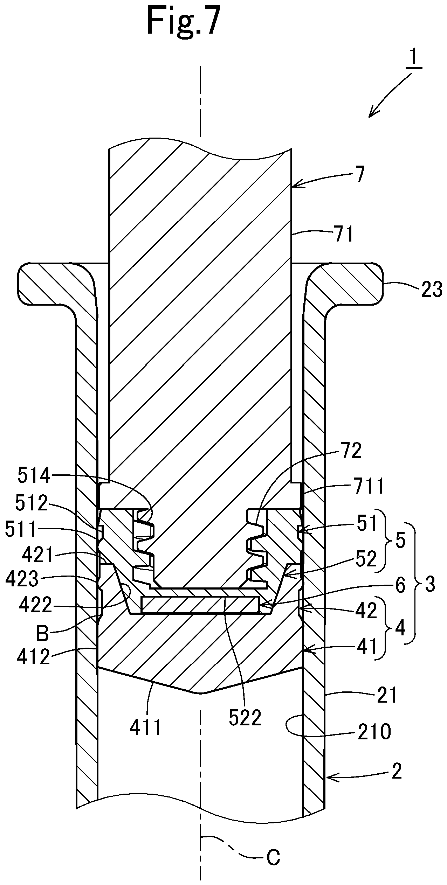

As shown in Fig. 7, the gasket 3 with the RF tag 6 therein is disposed in the barrel 21, and in this state, the male screw part 72 of the plunger rod 7 is screwed into the screw hole 514, that is, the gasket 3 is connected to the distal end of the plunger rod 7. At this time, the gasket 3 has a larger diameter than the inner diameter of the barrel 21 at the large diameter part 41, the first annular projection 423, and the second annular projection 511 (see Fig. 2), and thus is held in a compressed state in the radial direction in the barrel 21. The elastic force due to this compression brings the large diameter part 41 and the annular projections 423, 511 into close contact with the entire area of the inner peripheral surface 210 of the barrel 21 in the circumferential direction, thereby ensuring the liquid-tightness of the syringe 1 between the barrel 21 and the gasket 3.

Thus, it is possible to ensure ease of elastic deformation (i.e., flexibility) of the large diameter part 41 in the radial direction by the syringe 1 described above, in which the RF tag 6 is disposed at a position (i.e., between the recess 422 of the first member 4 and the projection 52 of the second member 5) displaced in the direction of the central axis C relative to the large diameter part 41 of the gasket 3, the large diameter part 41 being configured to be elastically deformed in the radial direction in the barrel 21 to come into close contact with the inner peripheral surface 210 of the barrel 21. It is also possible to ensure ease of elastic deformation of the part in which two members overlap with each other in the gasket 3 in the radial direction (i.e., part in which the projection 52 of the second member 5 fits into the recess 422 of the first member 4) by the configuration where the second member 5 is provided with the screw hole 514 extending in the direction of the central axis C to the inside of the projection 52, which minimizes the thickness of the aforementioned part. These configurations can suppress the sliding resistance when the gasket 3 is made to slide in the barrel 21. Further, it is possible to suppress the deformation of the RF tag 6 by the configuration where the RF tag 6 is disposed between the recess 422 of the first member 4 and the projection 52 of the second member 5, which can reduce the pressure on the RF tag 6 in the radial direction when the gasket 3 is disposed in the barrel 21.

Also, according to the syringe 1 of this embodiment, the small diameter part 42 of the gasket 3 includes the first annular projection 423 that projects radially outward at a position with a distance from the large diameter part 41 in the direction of the central axis C and extends through the entire area in the circumferential direction, and the first annular projection 423 is in close contact with the entire area of the inner peripheral surface 210 of the barrel 21 in the circumferential direction when the gasket 3 is inserted into the barrel 21. Further, according to the gasket 3, the RF tag 6 is disposed between the large diameter part 41 and the first annular projection 423 in the direction of the central axis C, and the screw hole 514 extends to the position of the first annular projection 423 in the direction of the central axis C. As described above, the gasket 3 in a state of being inserted in the barrel 21 is in close contact with the inner peripheral surface 210 of the barrel 21 at two positions with a distance from each other in the direction of the central axis C (i.e., the position of the large diameter part 41 and the position of the first annular projection 423), and thus the liquid-tightness between the barrel 21 and the gasket 3 is more sufficiently ensured. Moreover, it is possible to effectively suppress increase in the sliding resistance due to the increase in parts in close contact with the inner peripheral surface 210 of the barrel 21 by the configuration where the screw hole 514 in the gasket 3 extends to the position at which the first annular projection 423 is formed to thereby suppress the thickness of this part (that is, the ease of elastic deformation of this part can be ensured).

In the syringe 1 of this embodiment, it is possible to further ensure the liquid-tightness between the barrel 21 and the gasket 3 by the configuration where the second annular projection 511 located at the position with a distance from the first annular projection 423 in the direction of the central axis C is also in close contact with the inner peripheral surface 210 of the barrel 21. Also, it is possible to suppress the sliding resistance due to the increase in parts of the gasket 3 in close contact with the inner peripheral surface 210 of the barrel 21 by the configuration where the screw hole 514 is provided to extend in the direction of the central axis C to the position at which the second annular projection 511 is provided , and thus, the ease of elastic deformation in this part can be ensured in the same manner as the first annular projection 423,.

Moreover, in the syringe 1 of this embodiment, it is possible to suppress Thus, the increase in the sliding resistance due to the increase in parts of the gasket 3 in close contact with the peripheral surface 210 of the barrel 21 by the configuration where a gap that is formed between the inner peripheral surface of the screw hole 514 and the male screw part 72 of the plunger rod 7, which can ensure ease of deformation at the position at which the first annular projection 423 of the gasket 3 is provided.

The syringe gasket and the syringe including the syringe gasket of the present invention are not limited to the aforementioned embodiments. For example, a configuration of another embodiment can be added to a configuration of one embodiment, and a part of the configuration of one embodiment can be replaced with the configuration of the other embodiment. Further, a part of the configuration of one embodiment can be omitted.

In the gasket 3 of the above embodiment, the RF tag 6 is disposed inside the gasket 3, but is not limited to this configuration. The member to be disposed inside the gasket 3 is not limited to the RF tag 6, and any member can be employed as long as an member can allow information to be remotely read out and written in (that is, from the outside of the gasket 3 or from the outside of the syringe 1). Also, the member disposed inside the gasket 3 can be an element capable of sensing, for example, temperature and pressure, and capable of remotely transmitting the results of the sensing.

The element (RF tag) 6 of the above embodiment has a higher hardness than the first member 4 and the second member 5 of the gasket 3, but is not limited to this configuration. The hardness of the element 6 can be the same as or lower than the hardness of the first member 4 and the second member 5. The element 6 of the above embodiment has a plate shape extending in the direction orthogonal to the central axis C, but is not limited to this shape. Any shape can be adopted as long as the element 6 can be disposed inside the gasket 3.

The element (RF tag) 6 of the above embodiment is not adhered (joined) to either the first member 4 or the second member 5, but is not limited to this configuration. For example, it can be configured such that the distal side surface 61of the element (RF tag) 6 is adhered to the first member 4 (specifically, the bottom surface 4221 of the recess 422) via an adhesive layer, and a proximal side surface 62 of the element (RF tag) 6 is adhered to the second member 5 (specifically, the bottom surface 5221 of the distal end recess 522) via an adhesive layer. In this case, the entire area or part of the area of each of the surfaces 61, 62 can be adhered to the first member 4 or the second member 5 in the element (RF tag) 6. Since both the surfaces 61, 62 of the plate-shaped RF tag 6 are adhered to the first member 4 and the second member 5 in this manner, the variation of the sliding resistance in each syringe 1 (i.e., sliding resistance during the movement of the plunger rod 7 in the direction of the central axis C relative to the syringe body 2) can be suppressed.

In addition to the RF tag (element) 6, the gasket 3 of the above embodiment is composed of two members having elasticity (i.e., the first member 4 and the second member 5) aligned in the direction of the central axis C, but is not limited to this configuration, and can be composed of three or more members having elasticity aligned with each other in the direction of the central axis C.

The distal end surface 411 of the gasket 3 of the above embodiment has a conical shape, but is not limited to this configuration. The distal end surface 411 can be a surface extending in the direction orthogonal to the central axis C.

In the gasket 3 of the above embodiment, the screw hole 514, into which the male screw part 72 of the plunger rod 7 is screwed, extends in the direction of the central axis C from the proximal side surface 513 of the gasket 3 to the position of the first annular projection 423, but is not limited to this configuration. The screw hole 514 can extend to a certain position on the distal side of the position of the first annular projection 423, and can extend to a certain position on the proximal side of the position of the first annular projection 423, in the direction of the central axis C.

In the gasket 3 of the above embodiment, the first member 4 and the second member 5 are made of the same material without limitation thereto. The first member 4 and the second member 5 can be made of different materials, respectively.

In the gasket 3 of the above embodiment, the outer diameter ϕ4 of the part in which the first annular projection 423 is provided and the outer diameter ϕ6 of the part in which the second annular projection 511 is provided are the same or substantially the same as each other without limitation thereto. One of the outer diameter ϕ4 and the outer diameter ϕ6 can be larger than the other one within a range not exceeding the outer diameter ϕ2 of the large diameter part 41.

The nominal volume of the syringe body 2 of the above embodiment is 5 mL, but any volume can be adopted. The nominal volume can be, for example, from 0.5 mL to 100 mL. In this case, the barrel 21 is configured to have an inner diameter corresponding to each volume, and each of the diameters in the gasket 3 is also configured to have a size corresponding thereto.

The gasket 3 of the above embodiment is formed such that a second molding is made on the RF tag 6 disposed in the recess 422 (on the bottom surface 4221) of the first member 4 that is fitted into the cavity 811 of the mold 8 (lower mold 81). Thereby, a product (gasket 3) is molded. That is, the distal end recess 522 of the second member 5 is inevitably formed by the shape of the RF tag 6 at the time of the second molding (i.e., at the time of molding of the second member 5) without limitation thereto. The configuration can be such that, for example, the projection 52 without a recess is molded at the time of molding of the second member 5, and then the distal end recess 522 is molded, that is, the first member 4 and the second member 5 are separately molded and then connected to each other.

The configuration can be such that the first member 4 and the second member 5 are separately molded, and then the first member 4 and the second member 5 are joined to each other so as to locate the RF tag 6 between the recess 422 of the first member 4 and the projection 52 of the second member 5.

The present invention has been appropriately and sufficiently described as above through embodiments with reference to the drawings in order to express the present invention, but it shall be recognized that those skilled in the art could easily modify and/or improve the aforementioned embodiments. Therefore, it shall be construed that any modified embodiment or improved embodiment by those skilled in the art is covered by the scope of the claims unless they depart from scope of the claims.

### REFERENCE SIGNS LIST

1: Syringe
2: Syringe body
21: Barrel
210: Inner peripheral surface
22: Discharge part
23: Flange
3: Gasket (syringe gasket)
4: First member
41: Large diameter part
411: Surface (distal end surface)
412: Peripheral surface
42: Small diameter part
421: End surface (end surface on the proximal side)
422: Recess
4221: Bottom surface
4222: Inner peripheral surface
423: First annular projection
5: Second member
51: Base part
511: Second annular projection
512: Annular recess
513: End surface (end surface on the proximal side)
514: Screw hole
5141: Bottom surface
515: Distal end surface
52: Projection
520: Distal end surface
521: Outer peripheral surface
522: Distal end recess
5221: Bottom surface
5222: Inner peripheral surface
6: RF tag (element)
61: Distal side surface
62: Proximal side surface
7: Plunger rod
71: Rod body
711: Pressing part
72: Male screw part
73: Flange
8: Pair of mold
81: Lower mold
81a: Upper end surface
811: Cavity
82: Upper mold
82a: Lower end surface
821: Projection
B: Boundary position
C: Central axis
K: Virtual surface
S: Space
S1: Area
T1: Thickness of the large diameter part
T2: Thickness of the small diameter part
T3: Thickness of the base part
T4: Thickness of the element
α1: Inclination (angle) of the inner peripheral surface of the recess of the first member relative to the central axis
ϕ1: Inner diameter of barrel
ϕ2: Outer diameter of the large diameter part of the first member
ϕ3: Outer diameter of the small diameter part
ϕ4: Outer diameter of the part in which the first annular projection in the small diameter part is provided
ϕ5: Outer diameter of the base part of the second member
ϕ6: Outer diameter of the part in which the second annular projection in the base part of the second member is provided
ϕ7: Inner diameter of the distal end recess
ϕ8: Outer diameter of the RF tag

## Claims

1. A syringe gasket (3) configured to be connected to a distal end of a plunger rod (7), while being disposed in a cylindrical barrel (21), the syringe gasket comprising:
a first member (4) having a large diameter part and a small diameter part, the large diameter part being in close contact with an entire area of an inner peripheral surface of the barrel in a circumferential direction when the syringe gasket is inserted into the barrel, the small diameter part being located adjacent to the large diameter part and having a smaller diameter than the large diameter part;
a second member (5) configured to be connected to the first member from a side of the small diameter part in an axial direction that is a direction in which the large diameter part and the small diameter part are aligned with each other; and
an element (6) disposed between the first member and the second member,
the first member and the second member having elasticity,
the first member having a recess (422) that is recessed from an end surface on a side of the second member toward the large diameter part in the axial direction,
the recess having a bottom that is recessed to reach a boundary position between the large diameter part and the small diameter part in the axial direction or a certain position on the side of the second member relative to the boundary position,
the second member having a projection (52) that projects toward the first member and is configured to fit into the recess with the element disposed between the recess and the projection, and having a screw hole (514) extending from an end surface of the second member on an opposite side to the first member toward the first member to reach an inside of the projection in the axial direction to allow a screw part (72) provided at the distal end of the plunger rod to be screwed thereinto.

2. The syringe gasket according to claim 1, wherein
the small diameter part has an annular projection that projects radially outward with a distance from the large diameter part in the axial direction, while extending through the entire area in the circumferential direction,
the annular projection is in close contact with an entire area of the inner peripheral surface of the barrel in the circumferential direction when the syringe gasket is inserted into the barrel,
the element is disposed between the large diameter part and the annular projection in the axial direction, and
the screw hole extends at least to a position of the annular projection in the axial direction.

3. A syringe comprising:
a syringe body having a cylindrical barrel:
the syringe gasket according to claim 1 or 2 being disposed in the barrel; and
a plunger rod extending along a central axis of the barrel and having a screw part that is provided in a distal end of the plunger rod and configured to be threaded into the screw hole of the syringe gasket.

## Patentansprüche

1. Spritzendichtung (3), die dafür ausgelegt ist, mit einem distalen Ende einer Stößelstange (7) verbunden zu sein, während sie in einer zylindrischen Hülse (21) angeordnet ist, wobei die Spritzendichtung aufweist:
ein erstes Bauteil (4), das einen Teil mit großem Durchmesser und einen Teil mit kleinem Durchmesser hat, wobei der Teil mit großem Durchmesser in einer Umfangsrichtung in engem Kontakt mit dem gesamten Bereich einer Innenumfangsfläche der Hülse ist, wenn die Spritzendichtung in die Hülse eingeführt ist, wobei sich der Teil mit kleinem Durchmesser angrenzend an den Teil mit großem Durchmesser befindet und einen kleineren Durchmesser als der Teil mit großem Durchmesser hat;
ein zweites Bauteil (5), das dafür ausgelegt ist, mit dem ersten Bauteil von einer Seite des Teils mit kleinem Durchmesser her in Axialrichtung verbunden zu sein, die eine Richtung darstellt, in welcher der Teil mit großem Durchmesser und der Teil mit kleinem Durchmesser miteinander ausgerichtet sind; und
ein Element (6), das zwischen dem ersten Bauteil und zweiten Bauteil angeordnet ist,
wobei das erste Bauteil und das zweite Bauteil über Elastizität verfügen,
wobei das erste Bauteil eine Aussparung (422) hat, die von einer Stirnfläche an einer Seite des zweiten Bauteils in Axialrichtung zu dem Teil mit großem Durchmesser hin ausgespart ist,
wobei die Aussparung einen Boden hat, der ausgespart ist, um in Axialrichtung eine Grenzposition zwischen dem Teil mit großem Durchmesser und dem Teil mit kleinem Durchmesser zu erreichen, oder um eine bestimmte Position an der Seite des zweiten Bauteils relativ zur Grenzposition zu erreichen,
wobei das zweite Bauteil einen Ansatz (52) hat, der zum ersten Bauteil hin vorsteht und dafür ausgelegt ist, sich in die Aussparung einzupassen, wobei das Element zwischen der Aussparung und dem Ansatz angeordnet ist, und eine Schraubenöffnung (514) hat, die sich von einer Stirnfläche des zweiten Bauteils auf einer zum ersten Bauteil entgegengesetzten Seite zum ersten Bauteil hin erstreckt, um einen Innenraum des Ansatzes in Axialrichtung zu erreichen, damit ein am distalen Ende der Stößelstange vorgesehener Schraubenteil (72) darin eingeschraubt werden kann.

2. Spritzendichtung nach Anspruch 1, wobei
der Teil mit kleinem Durchmesser einen ringförmigen Ansatz hat, der mit einem Abstand von dem Teil mit großem Durchmesser in Axialrichtung radial nach außen vorsteht, während er sich über den gesamten Bereich in Umfangsrichtung erstreckt,
wobei der ringförmige Ansatz mit dem gesamten Bereich der Innenumfangsfläche der Hülse in Umfangsrichtung in engem Kontakt ist, wenn die Spritzendichtung in die Hülse eingeführt ist,
wobei das Element in Axialrichtung zwischen dem Teil mit großem Durchmesser und dem ringförmigen Ansatz angeordnet ist und
die Schraubenöffnung sich in Axialrichtung mindestens bis zu einer Position des ringförmigen Ansatzes erstreckt.

3. Spritze, aufweisend:
einen Spritzenkörper, der eine zylindrische Hülse hat;
die Spritzendichtung nach Anspruch 1 oder 2, die in der Hülse angeordnet ist; und
eine Stößelstange, die sich entlang einer Mittelachse der Hülse erstreckt und einen Schraubenteil hat, der an einem distalen Ende der Stößelstange vorgesehen und dafür ausgelegt ist, in die Schraubenöffnung der Spritzendichtung eingeschraubt zu werden.

## Revendications

1. Joint de seringue (3) configuré pour être relié à une extrémité distale d'une tige de piston (7), tout en étant disposé dans un tube cylindrique (21), le joint de seringue comprenant :
un premier élément (4) ayant une partie de grand diamètre et une partie de petit diamètre, la partie de grand diamètre étant en contact étroit avec une zone entière d'une surface périphérique intérieure du tube dans une direction circonférentielle lorsque le joint de seringue est inséré dans le tube, la partie de petit diamètre étant positionnée à côté de la partie de grand diamètre et ayant un diamètre plus petit que la partie de grand diamètre ;
un second élément (5) configuré pour être relié au premier élément à partir d'un côté de la partie de petit diamètre dans une direction axiale qui est une direction dans laquelle la partie de grand diamètre et la partie de petit diamètre sont alignées l'une avec l'autre ; et
un élément (6) disposé entre le premier élément et le second élément ;
le premier élément et le second élément ayant une certaine élasticité ;
le premier élément ayant un renfoncement (422) qui est renfoncé par rapport à une surface d'extrémité sur un côté du second élément en direction de la partie de grand diamètre dans la direction axiale ;
le renfoncement ayant un fond qui est renfoncé pour atteindre une position limite entre la partie de grand diamètre et la partie de petit diamètre dans la direction axiale ou une certaine position sur le côté du second élément par rapport à la position limite ;
le second élément ayant une saillie (52) qui ressort en direction du premier élément et est configurée pour s'ajuster dans le renfoncement avec l'élément disposé entre le renfoncement et la saillie, et ayant un trou de vis (514) s'étendant hors d'une surface d'extrémité du second élément sur un côté opposé par rapport au premier élément en direction du premier élément pour atteindre l'intérieur de la saillie dans la direction axiale afin de permettre à une partie de vis (72) prévue à l'extrémité distale de la tige de piston d'être vissée à l'intérieur.

2. Joint de seringue selon la revendication 1, dans lequel :
la partie de petit diamètre a une saillie annulaire qui ressort vers l'extérieur dans le plan radial à une certaine distance par rapport à la partie de grand diamètre dans la direction axiale, tout en s'étendant à travers la zone entière dans la direction circonférentielle ;
la saillie annulaire est en contact étroit avec une zone entière de la surface périphérique intérieure du tube dans la direction circonférentielle lorsque le joint de seringue est inséré dans le tube ;
l'élément est disposé entre la partie de grand diamètre et la saillie annulaire dans la direction axiale ; et
le trou de vis s'étend au moins en direction d'une position de la saillie annulaire dans la direction axiale.

3. Seringue comprenant :
un corps de seringue ayant un tube cylindrique :
le joint de seringue selon la revendication 1 ou 2 étant disposé dans le tube ; et
une tige de piston s'étendant le long d'un axe central du tube et ayant une partie de vis qui est prévue dans une extrémité distale de la tige de piston et configurée pour être filetée dans le trou de vis du joint de seringue.
